# EUROPEAN PATENT APPLICATION

(11) **EP 1 912 063 A1**
(43) Date of publication of application: **16.04.2008**
(21) Application number: 07118181.2
(22) Date of filing: 10.10.2007
(51) Int. Cl.: G01N 27/22

(54) **Analysis and conditioning system of granular plastic material**

(30) Priority: 10.10.2006 IT PD20060375
(71) Applicant: Tibi S.p.A., 30035 Ballo' di Mirano (IT)
(72) Inventor: Targa, Rino, 30036 Santa Maria di Sala (IT)
(74) Representative: Borsano, Corrado

(57) **Abstract**

We have given an analysis and conditioning system of a plastic material in the sector of injection, extrusion or blowing of technopolymers. The system includes a sampling or storage area of the plastic material, where means of dehumidification, and a utilization area of the conditioned plastic material such as a processing machine or similar are included, and it is characterized by the fact that it includes means of continuous analysis in continuo (A) disposed in series between the sampling area and the utilization area, and a management remote unit capable of controlling together with these means of analysis (A).

## Description

### FIELD OF THE INVENTION

This invention refers to an analysis and conditioning system of a plastic material intended to be used in the sector of technopolymers injection, extrusion or blowing.

### STATE OF THE TECHNIQUE

It is common knowledge that the use of hygroscopic technopolymers (PA, PET, PEEK, ABS, ABS/PC, PMMA etc...) in injection, extrusion or blowing sectors is made possible only after the dehumidification of the plastic granules before the transformation. In effect, without dehumidification, the quality of the produced pieces would be partially or even completely compromised.

More precisely, the presence of water inside plastic during the melting process, engenders chemical and mechanical phenomena which can modify the molecular structure of the plastic material and make many of its qualities/properties decay. From these modifications, some defects of several kinds may be found on the produced pieces:
- Visual defects: stripes, opaque spots, black dots, blisters, fusions. For example, a bottle made up of P.E.T cannot be produced if the content of humidity of the plastic it is made up of is higher than 100 ppm (it would explode at the time of blowing).
- Invisible: loss of viscosity of the melted material (molecules broken by oxidation), irregular filling of the mould.
- Mechanical: structural weakening of the produced pieces, inconstant thicknesses, weakened resistance to tensile stress and pressure.

Therefore, the presence of water in plastic granules does not permit the correct use of the qualities of the plastic material.

Moreover, there is another problem linked to the possible excessive presence of heat in the plastic material, with consequent oxidation of the latter, with consequent degradation. This phenomenon can be easily observed during the dehumidification of nylon (PA): after the six hours' dehumidification, the PA material, for example, turns yellow and loses viscosity at the same time, with consequent impossibility to plasticize it correctly.

At present, several methods of dehumidification are known, which use alternatively microwaves dehumidifiers, infrared dehumidifiers, vacuum dehumidifiers, or dry air dehumidifiers.

The most used principle is dry air (80% of the existing dehumidifiers). The principle of these dehumidifiers is based on the removal of humidity from the plastic granule when in touch with dry air.

The owner of this demand of patent produces at present this type of process in its dehumidifiers called DET, and the system of this invention will be applied to these dehumidifiers.

For a correct dry air dehumidification in each plastic material, the parameters which have to be considered are :
- Dehumidification temperature, which can be easily checked through thermoregulators;
- Air flow (proportional to the kg/h of material to be dehumidified): the quantity of air is produced by the blowers used to carry the dry air in the dehumidification process. The engendered flow air depends on the pressure drop due to the state of obstruction of the process filters, and on the granulometry of the material;
- Dew-point of the air used to dehumidify, which is controlled through the use of a sensor (Dew-Point o Frost point Meter). The sensor of Dew-Point reacts to variation with a delay time of +/- 30 min.;
- Dehumidification time: it depends on the type of material to process which determines its permanence in the dehumidification hopper. The time of permanence of the material in the dehumidification hopper depends on the volume of the hopper and on the constancy of production in terms of kg/h.

As most of the parameters to be considered can be disturbed by the general process conditions, it has been and it is very common to oversized dehumidification plant with consequent waste of energy, due to over-dehumidification and sometimes with consequent degradation of plastic material.

### SUMMARY OF THE INVENZIONE

The purpose of this invention is to solve the inconveniences mentioned above by carrying out an analysis and conditioning system of granular plastic material in which a precision check of the requested dehumidification level is foreseen, according to the survey of humidity in real time by carrying out a setting of the DET, in order to reduce the power consumption of the dehumidifier, to guarantee a constant quality of the produced pieces and to eliminate the production waste.

### SHORT DESCRIPTION OF FIGURES

Now we are going to give a detailed description of a favourite form of carrying out of the analysis and conditioning system of granular plastic material of this invention, which will be given as an example and not as a restrictive description, referring to the attached pictures, in which:
figures 1, 2 and 3 show schematically a component of the analysis system of this invention according to three different forms of assembly configuration on plastic material conditioning plant;
figure 4 shows schematically the component of the analysis system of figure 1 according to a fourth assembly configuration on plastic material conditioning plant;
figure 5 shows schematically the component of the analysis system of figure 1 according to a fifth assembly configuration on plastic material conditioning plant;
figure 6 shows schematically the component of the analysis system of figure 1 according to a sixth assembly configuration on plastic material conditioning plant;
figure 7 shows schematically the component of the analysis system of figure 1 according to a seventh assembly configuration on plastic material conditioning plant;
figure 8 shows schematically the component of the analysis system of figure 1 according to an eighth assembly configuration on plastic material conditioning plant;
figure 9 shows schematically the component of the analysis system of figure 1 according to a ninth assembly configuration on plastic material conditioning plant;
figure 10 shows schematically the component of the analysis system of figure 1 according to a tenth assembly configuration on plastic material conditioning plant;
figure 11 shows schematically the component of the analysis system of figure 1 according to an eleventh assembly configuration on plastic material conditioning plant;
figure 12 shows schematically the component of the analysis system of figure 1 according to a twelfth assembly configuration on plastic material conditioning plant; and
figure 13 is a flow chart which shows the working logic of the analysis and conditioning system of plastic material of this invention.

### DETAILED DESCRIPTION OF THE INVENTION

Now we are going to explain the working principle of the analysis and control system of plastic material of this invention.

According to this invention, the plastic material to be dehumidified passes typically through a dehumidification hopper. In the dehumidification hopper the material collides with the dry air produced by the dehumidifier in closed circuit. On contact with the humid material, the dry air fills with water and goes back to the dehumidifier. The water contained in the coming-back air is released in molecular filters made up of materials in the form of granules, of porous crystalline structure with regular porosity of 10 Angstroms (the water molecule of diameter 4 Angstroms penetrates the channels of the crystal and is held due to polarity), the air, which is dry again, is once more sent in the dehumidification hopper to remove the water contained in the plastic material to be dehumidified. This process is continuous.

When the molecular filters are full of water, they have to be regenerated. The necessity of a continuous production process implies the presence of two filtering elements, one of them being processed and the other in regeneration or while waiting for the process. During the phase of regeneration of the molecular filters, they have to be isolated from the process and heated at about 300°C in order to have the water molecules vibrate and to free them from the channels of the porous crystals.

According to this invention, the system intends to apply (by interposition) a humidity analyzer in any point where there is a flow of plastic material outlined by a sampling (or storage) area to a utilization area, so applied in any point of the material line where it is necessary to monitor the humidity present in the plastic granule before entering the inlet of the processing machine (i.e. a vertical and horizontal injection press, an extrusor, a blower, etc.....).

The transport of the plastic material from a sampling or storage area to a utilization area can be carried out through suction, vacuum, thrust, drop, with mechanical devices (i.e. conveyer belts or Archimedes' screws), or manual containers (a simple bucket).

On the transport line of the plastic material (obviously before entering the inlet of the processing machine), systems of processing of the raw materials, or of storage, or of containment, or of maintenance can be interposed such as: driers, bins, insulated or not maintenance hoppers, storage containers, simple containers, containment and intake valves, rigid (steel, plastic materials, teflon, etc.....) or flexible (polycarbonate, stratified material, aluminium, etc.....) simple piping, in a new (in new plant), old (in already existing plant), special (on the customer's demand) context.

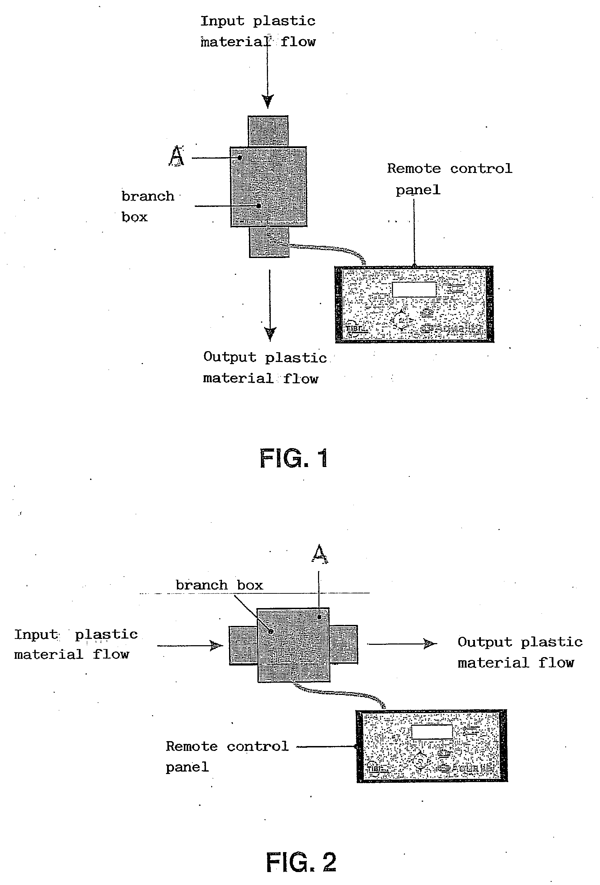

Referring particularly to figures 1, 2 and 3, they show schematically the disposition of a component A - humidity analyzer according to the system of this invention, as the analyzer A is aimed at checking the humidity present in the plastic material to be processed and as it is placed on a feeding line of the plastic material.

According to the system of this invention, the analyzer can be of the type described in the Patent for Industrial Invention n° IT1298821.

More specifically, the analyzer device has a substantially tubular structure and can be connected to the feeding piping of the plastic material to be analyzed, so that the flow of plastic material can pass through both the piping and the analyzer device. The checking of the humidity is done by passing through the plate of a measure condenser disposed coaxially with the body of the analyzer. The flow of plastic material, passing through the condenser without touching directly the plates makes the impedance of the condenser vary, causing a variation of its measure signal which is proportional to the quantity of humidity in the plastic material.

The checking of the measure signal permits to establish the content of humidity in the plastic material present in a transport flow which has to be analysed. The signal is checked and managed by an external control unit which manages also the whole analysis and conditioning process of the plastic material.

Therefore, according to this invention system, a new parameter for the dehumidification process is introduced: the checking of the real humidity (obtained from the dehumidification thanks to the particular disposition ed interface of the analyzer A with the remote unit) which permits a continuous feeding of the management and control system by fixing a highest level (too high level of humidity) and a lowest level (too low level humidity) of water content. In this way, the dehumidification process is automatically set on an intermediate optimal level for production, that is, when the material is too humid all the power of the dehumidifier is activated, whereas when the material is too dry part or all the power of the dehumidifier is deactivated.

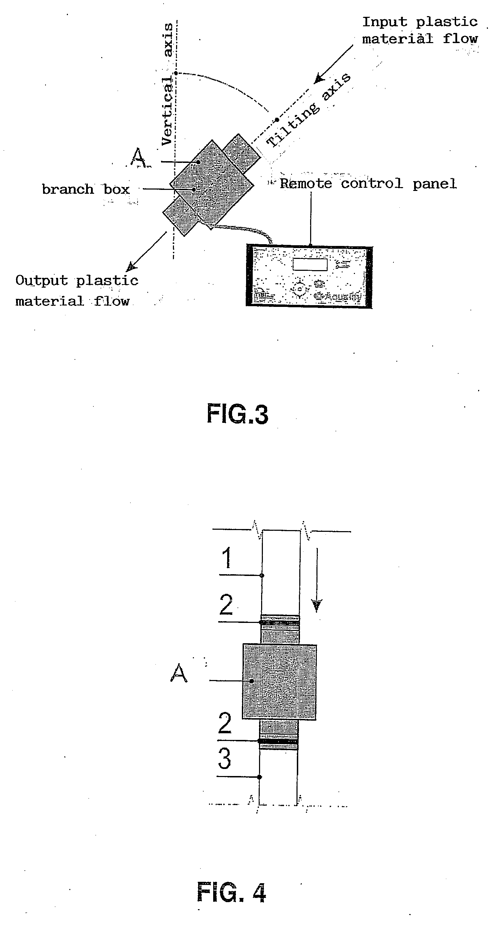

Figure 1 shows the disposition in a vertical application of the analyzer A with entrance and exit of the material flow. Its installation is reversible. Figure 2 shows the horizontal disposition of the analyzer A, while figure 3 shows la inclined disposition of the analyzer A. When it is inclined, the analyzer A has always one entrance and one exit of the material flow but the material flow has to be both constant and flowing. Its installation is reversible.

Figure 4 shows the application of the analyzer A on rigid or flexible tubes both in entrance and exit in a centralized plant or system. According to this application, the analyzer A è placed on the material flow of already existing plant or centralized system, or new or customized. The plastic material passes through the tube 2 which can be rigid or flexible, passes through the analyzer A and through the following tube 3 which can be rigid or flexible, to continue the working path. The analyzer A is installed mechanically through bands o collars, o connection muffs 3.

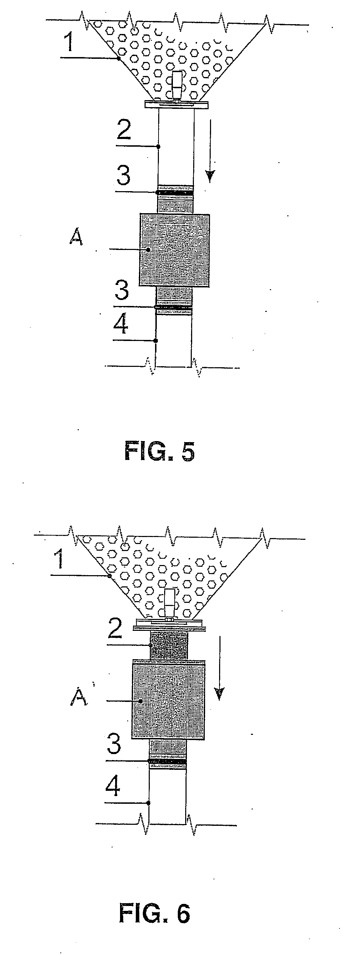

Figure 5 shows the application of the analyzer A on rigid or flexible tubes both in entrance and exit. In this application, the analyzer A is placed on the material flow coming from a device 1 as a dryer, bins, storage container, simple hoppers insulated or not, hoppers for the processing (dehumidification, drying, mixing, measuring, etc.....), chrystallizers, the material passes through the tube 2 which can be rigid or flexible, goes through the analyzer and through the following tube 4 which can be rigid or flexible, to continue the processing cycle.

The analyzer A is installed mechanically through bands o collars, o connection muffs 3.

Figure 6 shows the application of the analyzer A on an extender flange in entrance and rigid or flexible tube in exit. In this application, the analyzer is placed on the material flow coming from one of the following devices 1: dryer, bins, storage container, simple hoppers insulated and not, processing hoppers (dehumidification, drying, mixing, measuring, etc.), chrystallizers, so the material passes through an expander flange 2 installed directly on the provenience waste piping, goes through the analyzer A and through the following tube 4 which can be rigid or flexible, to continue the processing cycle. The analyzer A is installed mechanically through expander flange 2 and band 3 to fix it on tube 4.

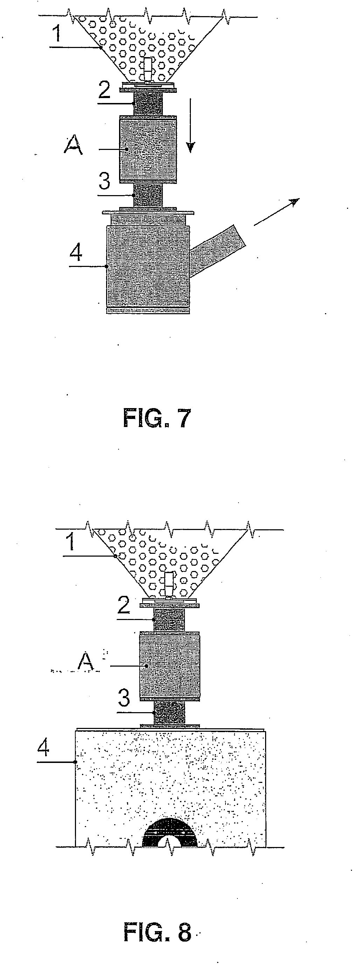

Figure 7 shows the application of the analyzer on expander flanges in entrance and exit on an exhaust or suction valve. In this application, the analyzer A è placed on the material flow coming from one of the following devices 1: dryer, bins, storage container, simple hoppers insulated or not, processing hoppers (dehumidification, drying, mixing, measuring, etc.....), chrystallizers. Therefore, the material passes through an expander flange 2 installed directly on the provenience waste piping, goes through the analyzer A and through the following expander flange 3 applied to any suction or exhaust valve 4 to continue the processing cycle. The analyzer A is installed mechanically through expander flanges 2 and 3.

Figure 8 shows the application of the analyzer A on expander flanges in entrance and exit, and in which it is placed on the material flow coming from one of the following devices 1: dryer, bins, storage container, simple hoppers insulated or not, processing hoppers (dehumidification, drying, mixing, measuring, etc.....), chrystallizers.

In this application, the plastic material passes through an expander flange 2 installed directly on the provenience waste piping, goes through the analyzer A and through the following expander flange 3 applied to any device 4 come: dryer, bins, storage container, simple hoppers insulated or not, processing hoppers (dehumidification, drying, mixing, measuring, etc.), chrystallizers to continue the processing cycle. The analyzer A is installed mechanically through expander flanges 2 and 3.

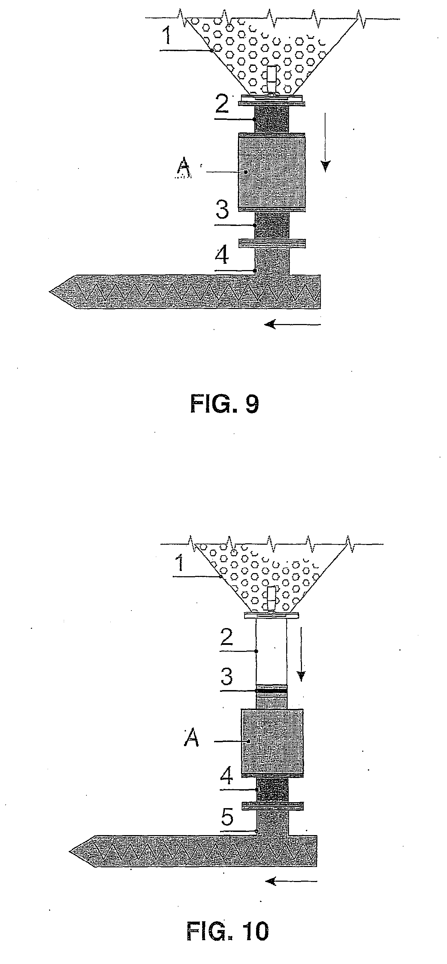

Figure 9 shows the application of the analyzer device A on expander flanges in entrance and exit directly on the inlet of a processing machine.

In this application, the analyzer A is placed on the material flow coming from a device 1 such as: dryer, bins, storage container, simple hoppers insulated or not, the material passes through an expander flange 2 installed directly on the provenience waste piping, goes through the analyzer A and through the following expander flange 3 applied directly on the inlet of any processing machine 4 such as: horizontal / vertical injection press, extrusor, blower, etc. to be transformed in a finished handmade article. The analyzer A is installed mechanically through expander flanges 2 e 3.

Figure 10 shows the application of the analyzer A on rigid or flexible tube in entrance and expander flange in exit directly on the inlet of a processing machine.

In this application, the analyzer A is placed on the material flow coming from a device 1 such as: dryer, bins, storage container, simple hoppers insulated or not, processing hoppers (dehumidification, drying, mixing, measuring, etc.), chrystallizers. Therefore, the material passes through tube 2 which can be rigid or flexible, goes through the analyzer A and through the following expander flange, directly applied on the inlet of any processing machine 5 such as: horizontal / vertical injection press, extrusor, blower, etc. to be transformed in a finished handmade article. The analyzer A is installed mechanically through expander flange 2 and band o collar, or connection muffs 3.

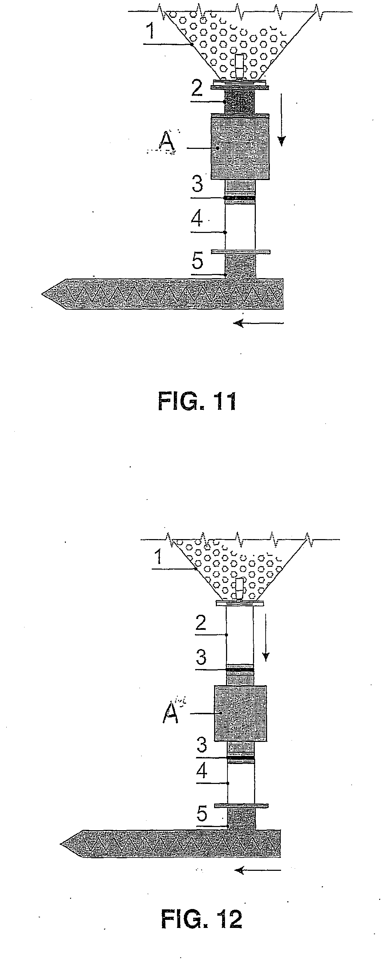

Figure 11 shows the application of the analyzer A on expander flange in entrance and rigid or flexible tube in exit directly on the inlet of the processing machine.

The analyzer A is placed on the material flow coming from a device 1 such as: dryer, bins, storage container, simple hoppers insulated or not, processing hoppers (dehumidification, drying, mixing, measuring, etc.), chrystallizers. The material passes through an expander flange 2 directly installed on the provenience waste piping, goes through the analyzer A and through the following tube 4 which can be rigid or flexible, directly applied on the inlet of any processing machine 5 such as: horizontal / vertical injection press, extrusor, blower, etc., to be transformed in finished handmade article. The analyzer A is installed mechanically through expander flange 2 e band o collars, or connection muffs 3.

Figure 12 shows the application of the analyzer A on rigid or flexible tubes in entrance and exit directly on the inlet of the processing machine.

According to this application, the analyzer A is placed on the material flow coming from a device 1 such as: dryer, bins, storage container, simple hoppers insulated or not, processing hoppers (dehumidification, drying, mixing, measuring, etc.....), chrystallizers. Therefore, the material passes through tube 2 which can be rigid or flexible, goes through the analyzer A and through the following tube 4 which can be rigid or flexible, directly applied on the inlet of any processing machine 5 such as: horizontal/vertical injection press, extrusor, blower, etc. to be transformed in a finished handmade article. The analyzer A is installed mechanically through bands or collars, or connecting muffs 3.

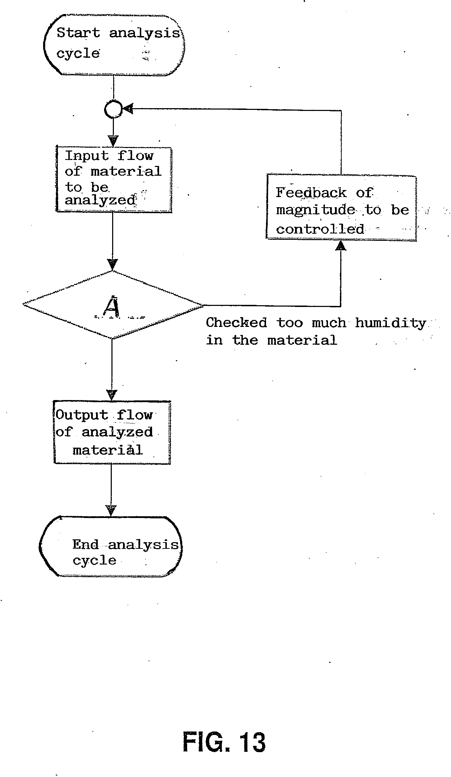

Referring to figure 13, it shows a flow of the working of this invention.

More precisely and according to this invention, the checking of humidity in real time (thanks to the disposition and to the interface of the analyzer A with the remote unit) permits a continuous feeding of the management and control system and control of all the preceding devices (dehumidifiers and not). After the setting of a highest figure of humidity (excessive humidity) and a lowest figure of humidity (too low level of humidity), the dehumidification process is automatically set on an intermediate optimal level for the production of the final handmade article.

The material coming from the feeder passes the analyzer and its humidity is checked; if it is too humid, all the power of the dehumidifier is activated until the set level is reached. On the other hand, if the checked humidity figure is lower than previously fixed (i.e. too dry) a part or all the power of the dehumidifier is deactivated, until the set level of humidity is reached.

The system of this invention has the following advantages:
- Constant quality of the produced pieces: dehumidifying and heating the material in a constant and repeatable way (according to our system) fixes the main parameters before processing. The production process is protected against environmental unforeseen events (temperature and humidity of the ambient air).
- Production long-term planning: when you are sure about the repeatable quality of the production, it is possible to plan more precisely the use of the processing machine (press, extrusor, blower).
- More rapid starting of the production: this system will be provided with an exit which will permit or not the extraction of the material only if it corresponds to the set figures of humidity and temperature. It is possible to know exactly when the material is dehumidified enough to start production without risks of machine stops or the production of waste pieces.
- Optimization of the production parameters: the fact of avoiding variations of humidity in entrance of the processing, permits a good regularity of the working and the possibility to optimize the settings of the processing machine. In this way, for example, it is possible to cool more quickly the pieces with consequent reduction of the cycle time and more productivity.
- Saving of raw materials: when designing the pieces, it is possible to set precisely the thickenings, reducing the quantity of plastic which is necessary to obtain the requested physical features.
- Increased possibility to use recycled material: if you know precisely the humidity of the materials to be transformed, it is possible to deduce its viscosity. If you have a certain viscosity, it is possible to determine with precision the quantity or percentage of ground material recoverable in the transformation process.
- Less waste of plastic additives: generally, additives are the first components which volatilize in case of over-dehumidification (plasticizing, anti-fire, catalyzers). Avoiding the degradation of the material due to over-dehumidification, it is possible to use all the active principles of additives without taking into consideration a part to be given up for drying problems.
- Correct measuring of the dehumidification system and of the preceding electrical protections: the possibility to choose the size of the dehumidifier according to real needs, without considering a margin of risk (up to now the average margin of risk foreseen by the dehumidifiers producers is of about 30%), permits to reduce as a consequence the power of the dehumidifiers.
- Less room occupied by the dehumidifier in the factory: also as regards this aspect, not having an exaggerate-sized dehumidification system, permits to propose small-size machines.
- Average power saving of 30% on all the appliances: this saving is due to the lack of necessity of having an oversized dehumidification system.
- Control of the production lots: the monitoring of the humidity content of the raw materials permits to carry out also a control of the lots of the material being produced.

## Claims

1. Analysis and conditioning system of granular plastic material, including a sampling or storage area of said plastic material in which a dehumidification hopper, a dehumidifier in closed circuit and containing at least a filtering element, and a utilization area of the conditioned plastic material such as a processing machine or similar are included,
the system is **characterised by** the fact of including means of analysis in continuo (A) disposed in series between the sampling area and the utilization area, and a remote management unit to help control said means of analysis (A), said sampling area, and said utilization area; the disposition is such as to enable the means of analysis (A) to carry out a continuous analysis of a flow of plastic material which passes through said means of analysis (A).

2. Analysis and conditioning system of granular plastic material according to the preceding claim, in which the transport of the plastic material from the sampling or storage area to the utilization area can be carried out through one of the following ways:
- suction;
- vacuum;
- thrust;
- drop;
- mechanical devices (conveyer belts and Archimedes' screws), and
- manual containers.

3. Analysis and conditioning system of granular plastic material according to claims 1 or 2, including also processing systems of raw materials, or of storage, or of containing, or of maintenance in the area which follows said means of analysis (A) and which precedes the processing machine, said systems being chosen in the following group: driers, bins, maintenance hoppers insulated or not, storage containers, containers, containment and suction valves, and piping both rigid and flexible.

4. Analysis and conditioning system of granular plastic material according to claims 1, 2 or 3, in which the means of continuous analysis of the plastic material include a device (A) with a substantially tubular structure, which can be connected to a feed piping of the plastic material to be analysed, the disposition being such that the plastic material flow passes both through the piping and the analyzer device (A).

5. Analysis and conditioning system of granular plastic material according to the preceding claim, in which the analyzer (A) has a main body with a transit light for the plastic material to be analysed, and a plate of a measure condenser disposed coaxially with the main body; the disposition permits that the plastic material passes through the plate without touching it directly.

6. Analysis and conditioning system of granular plastic material according to claims 4 or 5, in which this analyzer (A) after the plastic material has passed in it, emits an electrical signal according to the variation of the impedance of the condenser, said signal being proportional to the quantity of humidity in the plastic material.

7. Analysis and conditioning system of granular plastic material according to any of the preceding claims, in which the remote management unit processes and manages the electrical signals coming from the analyzer (A).

8. Method of analysis and conditioning of granular plastic material in a system of transformation of the plastic material according to the preceding claims, in which the following continuous phases are included:
- to check in real time the humidity of the granular plastic material to be conditioned through means of analysis (A) disposed in series between a sampling or storage area of the plastic material, and a utilization area of the conditioned plastic material;
- to feed continuously this preceding area until the set levels of humidity of the plastic material have been reached and according to the level of humidity checked by the means of analysis (A);
- to set automatically the process on an intermediate level of humidity of the granular plastic material; this intermediate level is previously set and corresponds to an optimal level for the production of the final handmade article; and
- send this granular plastic material already conditioned to the utilization area of the plastic material.

9. Method of analysis and conditioning of granular plastic material according to the preceding claim, in which the phase of continuous feeding includes a continuous feeding of the management and control system by setting a highest level and a lowest level of water content of this plastic material.
